# EUROPÄISCHE PATENTSCHRIFT

(11) **EP 2 583 702 B1**
(45) Veröffentlichungstag und Bekanntmachung des Hinweises auf die Patenterteilung: **02.09.2015**
(21) Anmeldenummer: 11185969.0
(22) Anmeldetag: 20.10.2011
(51) Int. Cl.: A61M 1/36

(54) **Verfahren zum Beenden einer Hämodialyse**
Method for ending haemodialysis
Procédé destiné à terminer une hémodialyse

(43) Veröffentlichungstag der Anmeldung: 24.04.2013
(73) Patentinhaber: D_MED Consulting AG, 47800 Krefeld (DE)
(72) Erfinder: Breuch, Gerd, 53844 Troisdorf (DE); Biermann, Frank, 22455 Hamburg (DE); Yanagimoto, Yoji, 1030 Schaerbeek (BE)
(74) Vertreter: Patentanwälte ter Smitten Eberlein Rütten Partnerschaftsgesellschaft

(56) Entgegenhaltungen:
- EP-A1- 1 457 218
- EP-A2- 0 992 255
- DE-A1- 19 605 260
- US-A1- 2006 213 835
- US-A1- 2010 192 686

## Beschreibung

Die Erfindung bezieht sich auf ein Hämodialysegerät zum Betreiben eines Verfahrens zum Beenden einer Hämodialyse.

Ein typisches Hämodialysegerät weist eine Dialysatseite und eine Blutseite auf. Zu der Dialysatseite zählt eine Dialysewasser-Quelle, die Dialysewasser für die Herstellung des Dialysats zur Verfügung stellt, und zwar in Form von durch eine Wasseraufbereitungsanlage aufbereiteten Trinkwassers oder in Form eines Dialysewasser- Vorrats. In dem Hämodialysegerät wird das Dialysewasser durch Zugabe von Elektrolyten und Puffersubstanzen zum Dialysat. Ferner zählen die Dialysatkammer eines eine Membran aufweisenden Dialysators und eine Dialysatpumpe, die das Dialysat von der Dialysatquelle zu der Dialysatkammer pumpt, zu der Dialysatseite. Zwischen der Dialysewasser- Quelle und der Dialysatkammer ist eine Dialysatheizung vorgesehen, die das Dialysewasser oder das fertige Dialysat bei Bedarf ungefähr auf Körpertemperatur aufheizt. Schließlich ist auf der Dialysatseite zwischen der Dialysatkammer und einem Abfalltank eine Abfallpumpe vorgesehen. Zu der Blutseite zählen die Blutkammer des Dialysators, sowie eine arterielle Leitung und eine venöse Leitung, die beide an die Blutkammer angeschlossen sind. Die freien Enden der beiden blutseitigen Leitungen weisen Kanülen auf, die in Blutgefäße des Patienten eingesteckt werden können.

Beim Beenden der Hämodialyse- Behandlung, dem so genannten Ablegen, werden zunächst die beiden Leitungen der Blutseite, die den extrakorporalen Kreislauf bilden, mit Dialysat oder einer Kochsalzlösung gefüllt, das bzw. die auf diese Weise das Patientenblut zurück in die Blutgefäße des Patienten schiebt.

Aus DE 196 55 225 B4 ist bekannt, die beiden Leitungen symmetrisch und gleichmäßig mit Dialysat zu füllen, wobei das Dialysat von der Dialysatpumpe zum Dialysator gepumpt wird, und dort durch die Membran in die Blutkammer hindurchtritt. Die Dialysatheizung ist dabei abgeschaltet, so dass das Dialysat bezüglich seiner Eigentemperatur ungeregelt in den Dialysator bzw. in die extrakorporalen Leitungen gepumpt wird. Von der Blutkammer aus fließt das Dialysat in die beiden Leitungen und schiebt das Patientenblut in den Leitungen vor sich her zurück in den Patientenkörper, wobei auch temperaturungeregeltes Dialysat in den Patientenkörper gelangen kann.

US 2010/0192686 offenbart unter dem Begriff "rinse back" nach der eigentlichen Hämodialyse-Behandlung ein Rückspülen des Patientenblutes in den Patienten durch Füllen der Geräteleitungen mit Dialysat, das von der Dialysatpumpe durch den Dialysator in die Leitungen gepumpt wird, bis kein Patientenblut mehr in den Leitungen enthalten ist. Hierbei wird das Dialysat durch eine Heizung aufgeheizt.

US 2006/0213835 A1 offenbart ein Hämodialysegerät, das mehrere Drucksensoren aufweist, mit deren Hilfe ein Differenzdruck ermittelt werden kann. Mit Hilfe des Differenzdruckes kann während des sogenannten "primings" festgestellt werden, wann Blut den Dialysator erreicht.

Aus EP 1 457 218 A1 ist ein Hämodialysegerät bekannt, das eine Dialysatpumpe und eine Abfallpumpe aufweist, die zu einer Bilanzierpumpe gekoppelt sind.

Aufgabe der Erfindung ist es demgegenüber, ein Hämodialysegerät für ein Verfahren zum Beenden der Hämodialyse- Behandlung zu schaffen, bei dem beim Ablegen das Patientenblut zuverlässig und verträglich in den Patientenkörper zurückgepumpt wird.

Diese Aufgabe wird erfindungsgemäß gelöst mit den Merkmalen des Anspruchs 1.

Gemäß dem erfindungsgemäßen Hämodialysegerät ist verfahrensgemäß vorgesehen, während des Füllens der extrakorporalen Leitungen der Blutseite mit Dialysat das von der Dialysatpumpe zu der Dialysatkammer gepumpte Dialysat bzw. Dialysewasser durch eine Dialysat-Durchlaufheizung ungefähr auf Körpertemperatur aufzuheizen. Es ist unvermeidlich, dass beim Dialysatfüllen der extrakorporalen Leitungen auch gewisse Mengen Dialysat durch eine oder beide Leitungen in den Patientenkörper gepumpt werden, wenn sichergestellt werden soll, dass eine größtmögliche Menge an Patientenblut zurück in den Patientenkörper gepumpt wird. Es soll jedoch nach Möglichkeit verhindert werden, dass hierbei unterkühltes Dialysat in den Patientenkörper gepumpt wird. Dies wird erst dadurch zuverlässig vermieden, dass die Dialysat- Durchlaufheizung das zu dem Dialysator gepumpte Dialysat ungefähr auf Körpertemperatur aufheizt, so dass kein unterkühltes Dialysat in den Patientenkörper gelangen kann. Hierbei ist grundsätzlich unerheblich, ob die Dialysat-Durchlaufheizung das Dialysewasser, das den allergrößten Teil des Dialysats ausmacht, oder das fertige Dialysat erwärmt. Die Durchlaufheizung ist in der Lage, auch bei großen Dialysat-Flussraten das Dialysat auf die erforderliche Temperatur aufzuheizen.

Unter einer Hämodialyse ist vorliegend jede extrakorporale Blutbehandlung zu verstehen, also auch die durch ein Akutgerät.

Die Verbindung zwischen den blutseitigen Leitungen und dem Patlentenlcärper wird durch eine bzw. zwei Kanülen hergestellt, die in die Blutgefäße des Patienten münden. Der Innendurchmesser derartiger Kanülen ist relativ klein, so dass die Kanülen einen nennenswerten Flusswiderstand darstellen. Das Patientenblut weist eine erheblich höhere Viskosität auf als das Dialysat. Solange das Patientenblut durch die Kanülen hindurch in den Patientenkörper fließt, ist der Flusswiderstand größer als beim Hindurchfließen von Dialysat durch die Kanüle, so dass auch der von dem betreffenden Drucksensor gemessene Druck beim Hindurchfließen von Blut durch die Kanüle höher ist, also oberhalb einer festgelegten Dialysatdruckdurchtritts- Druckschwelle liegt, als beim Hindurchfließen von Dialysat. Sobald der Dialysatdruck also die festgelegte Dialysatdurchtritts-Druckschwelle dauerhaft durchschreitet bzw. unterschreitet, kann davon ausgegangen werden, dass das Patientenblut mehr oder weniger vollständig in den Patientenkörper zurückgepumpt wurde, und nur noch Dialysat aus den Leitungen durch die Kanüle(n) in den Patientenkörper nachfließt. Sobald ein Durch- bzw. Unterschreiten der Dialysatdurchtritts- Druckschwelle festgestellt wird, kann das Dialysatfüllen der Leitungen also mit einem Restvolumen volumengesteuert beendet werden. Dabei kann das noch zu pumpende festgelegte Restvolumen gleich Null sein, kann jedoch auch größer als Null sein.

Gemäß einer bevorzugten Ausgestaltung ist an der Leitung zwischen der Dialysat-Durchlaufheizung und dem Dialysator ein Temperatursensor angeordnet, der die Temperatur des vorbeifließenden Dlalysats ermittelt. Ferner ist ein Temperaturregler vorgesehen, der zusammen mit dem Temperatursensor und der Dialysat-Durchlaufheizung einen Regelkreis bildet, so dass die Temperatur des Dialysats auf einen konstanten Sollwert geregelt werden kann. Der Sollwert ist so gewählt, dass das Dialysat beim Austritt aus den extrakorporalen Leitungen der Blutseite und beim Eintritt in den Patientenkörper ungefähr Körpertemperatur aufweist.

Vorzugsweise ist im Verlauf der venösen Leitung eine Luftfalle vorgesehen, durch die Luftblasen in der zurück zum Patientenkörper fließenden Flüssigkeit zurückgehalten werden. Vor oder an der Luftfalle ist ferner ein Leitungszugang zu der arteriellen Leitung vorgesehen. Vor dem Starten des Dialysatfüllens wird das patientenseitige Ende der arteriellen Leitung an diesen Leitungszugang angeschlossen, so dass während des Dialysatfüllens nur noch die venöse Leitung unmittelbar an den Patientenkörper angeschlossen bleibt. Die arterielle Leitung dagegen mündet vor oder an der Luftfalle in die venöse Leitung, und zwar derart, dass auch die Luftblasen der aus der arteriellen Leitung abfließenden Flüssigkeit von der Luftfalle zurückgehalten werden können. Auf diese Weise wird sichergestellt, dass auch das Blut und das Dialysat aus der arteriellen Leitung vor Eintritt in den Patientenkörper frei von Luftblasen ist, ohne dass hierfür ein erhöhter konstruktiver Aufwand erforderlich ist. Gemäß einer hierzu alternativen Ausführungsform bleiben beide extrakorporalen blutseitigen Leitungen während des Dialysatfüllens jeweils mit ihren jeweiligen Kanülen unmittelbar mit dem Patientenkörper verbunden. Vor dem Dialysatfüllen ist also keinerlei Umstecken der extrakorporalen Leitungen erforderlich. Im Verlauf der arteriellen Leitung und im Verlauf der venösen Leitung ist jeweils ein Luftsensor vorgesehen, durch den Lufteinschlüsse bzw. Luftblasen in der durch die venöse und durch die arterielle Leitung fließenden Flüssigkeit zuverlässig detektiert werden können. Im Falle einer Detektion von Luft wird das Dialysatfüllen beispielsweise durch Schließen einer Schlauchklemme sofort gestoppt, bevor die Luft den Patientenkörper erreicht. Hierdurch wird sichergestellt, dass durch keine der beiden extrakorporalen Leitungen Luft in den Blutkreislauf des Patienten gelangen kann.

Vorzugsweise weist das Hämodialysegerät einen Anwesenheitsdetektor auf, der mit einer Gerätesteuerung verbunden ist. Die Gerätesteuerung steuert das Hämodialysegerät derart, dass das Dialysatfüllen sofort gestoppt wird, wenn und solange der Anwesenheitsdetektor manuell nicht gedrückt ist. Der Anwesenheitsdetektor kann ein mechanischer Taster sein, kann jedoch auch in Form eines virtuellen Tasters auf einem Touchscreen realisiert sein. Der Anwesenheitsdetektor kann auch ein Kartenleser sein, der die Anwesenheit einer Smart-Card oder eines RFID-Senders detektiert, oder kann ein Raumüberwachungssensor sein, der die Anwesenheit einer Person vor dem Hämodialysegerät feststellt. Durch den Anwesenheitsdetektor kann sichergestellt werden, dass während des Dialysatfüllens eine Bedienperson an dem Hämodialysegerät anwesend ist. Die Bedienperson kann durch Augenschein insbesondere kontrollieren, ob in den extrakorporalen Leitungen Luft oder Luftblasen vorhanden ist bzw. sind. Wenn dies der Fall ist, kann die Bedienperson das Dialysatfüllen sofort manuell stoppen, um auf diese Weise die Einleitung von Luft in den Blutkreislauf des Patienten zu verhindern. Dies ist insbesondere dann sinnvoll, wenn beim Dialysatfüllen die arterielle Leitung an den Patientenkörper direkt angeschlossen bleibt, da in diesem Fall die Flüssigkeit aus der arteriellen Leitung ohne Passieren einer Luftfalle und eines Luftsensors in dem Patientenkörper gepumpt wird.

Vorzugsweise erfolgt das Dialysatfüllen der Leitungen der Blutseite volumengesteuert, wobei das Dialysat-Füllvolumen größer ist als das Volumen der Dialysator- Blutkammer zuzüglich der Volumina der beiden Leitungen der Blutseite. Das Dialysat-Füllvolumen kann also so gewählt werden, dass auch unter ungünstigen Bedingungen sichergestellt ist, dass das gesamte Patientenblut aus den Leitungen in den Patientenkörper zurückgepumpt wird, also auch dann, wenn beispielsweise die Flusswiderstände und/oder die Gesamtvolumina der beiden Leitungen verschieden voneinander sind.

Gemäß einer bevorzugten Ausgestaltung ist zwischen der Dialysatquelle und der Dialysatkammer des Dialysators ein einziger Sterilisationsfilter vorgesehen. Ein Sterilisationsfilter in diesem Sinne ist ein Filter, der Viren, Bakterien, Endotoxine sowie Pilze annähernd vollständig zurückhält. Als zweiter Sterilisationsfilter, der eine Redundanz herstellt, dient die Dialysator- Membran, die aufgrund ihrer Feinporigkeit ebenfalls wie ein Sterilisationsfilter wirkt. Der Dialysator muss hierfür validiert sein, d.h. in Bezug auf den Rückhalt von Endotoxinen usw. geprüft und zusätzlich unmittelbar vor der Behandlung auf Dichtigkeit und Beschädigungsfreiheit getestet sein. Ein zweiter separater Sterilisationsfilter ist nicht erforderlich. Das Vorsehen eines Sterilisationsfilters ist vorliegend grundsätzlich erforderlich, weil beim Dialysatfüllen Dialysat in den Patientenkörper gelangen kann und weil das Dialysat unsteril sein kann. Letzteres kann insbesondere dann zutreffen, wenn das Dialysewasser aus aufbereitetem Leitungswasser hergestellt wird.

Gemäß einer bevorzugten Ausgestaltung weist das Hämodialysegerät einen Dialysat- Drucksensor zwischen der Dialysatkammer des Dialysators und der Abfallpumpe, einen arteriellen Drucksensor im Verlauf der arteriellen Leitung und/oder einen venösen Drucksensor im Verlauf der venösen Leitung auf. Während des Dialysatfüllens wird ständig der Fluiddruck durch den betreffenden Drucksensor bestimmt, wobei das Dialysatfüllen volumengesteuert beendet wird, nachdem der Fluiddruck eine festgelegte Dialysatdurchtritts- Druckschwelle durch- bzw. unterschreitet. Der Fluiddruck kann durch irgendeinen der drei Drucksensoren ermittelt und überwacht werden.

Vorzugsweise ist im Verlauf der venösen Leitung ein Farbsensor angeordnet, wobei das Dialysatfüllen volumengesteuert beendet wird, sobald der Farbsensor in der venösen Leitung Dialysat detektiert. Der Farbsensor kann als transmissiver oder reflexiver optischer Sensor ausgebildet sein, der bei einer oder mehreren Wellenlängen die Transmissivität der Flüssigkeit in der venösen Leitung detektiert. Mit dem Farbsensor kann zuverlässig die Ankunft des Dialysats detektiert werden. Nachdem die Dialysat-Ankunft auf diese Weise festgestellt wurde, kann das Dialysatfüllen der Leitungen mit einem Restvolumen volumengesteuert beendet werden. Dabei kann das noch zu pumpende festgelegte Restvolumen gleich Null sein, kann jedoch auch größer als Null sein. Auch die arterielle Leitung kann einen derartigen Farbsensor aufweisen.

Vorzugsweise wird die Blutpumpe während des Dialysatfüllens gemäß dem ersten Ausführungsbeispiel, bei dem beide Kanülen direkt an den Patientenkörper angeschlossen bleiben, ungefähr mit der Pumprate betrieben, die eine ungefähr gleichzeitigen Ankunft des Dialysats an den beiden Enden bzw. den beiden Kanülen der beiden blutseitigen Leitungen gewährleistet. Die Pumprate der Blutpumpe richtet sich also nach dem Verhältnis der Innenvolumina der arteriellen Leitung und der venösen Leitung. Wenn dieses Verhältnis exakt 50:50 ist, also die Volumina der venösen Leitung und der arteriellen Leitung gleich sind, beträgt die Pumprate der Blutpumpe exakt 50 % der Pumprate, mit der das Dialysat von der Dialysatkammer in die Blutkammer gepumpt wird.

Beim Dialysatfüllen gemäß dem zweiten Ausführungsbeispiel, bei dem die arterielle Leitung an einen Leitungszugang der venösen Leitung angeschlossen ist, ist die gleichzeitige Dialysatankunft an den beiden extrakorporalen Leitungsenden nicht erforderlich. Vielmehr kann bei dem zweiten Ausführungsbeispiel zunächst die arterielle Leitung vollständig mit Dialysat gefüllt werden, bis das gesamte Patientenblut aus der arteriellen Leitung in die venöse Leitung gepumpt ist. Erst anschließend wird ausschließlich die venöse Leitung mit Dialysat- gefüllt, bis die venöse Leitung kein Patientenblut mehr enthält. Alternativ können beide Leitungen gleichzeitig mit Dialysat gefüllt werden.

Die Blutpumpe ist einer der beiden blutseitigen Leitungen zugeordnet, und zwar in der Regel der arteriellen Leitung. Die Blutpumpe wird in diesem Fall beim Dialysatfüllen gemäß dem ersten Ausführungsbeispiel, bei dem beide Kanülen direkt an den Patientenkörper angeschlossen bleiben, in Gegenrichtung, also in umgekehrter Richtung als während der eigentlichen Dialyse und mit einer Pumprate betrieben, die ungefähr die gleichzeitige Ankunft des Dialysats an den freien Enden bzw. den Kanülen der beiden blutseitigen Leitungen ermöglicht. Bei dem Dialysatfüllen gemäß dem zweiten Ausführungsbeispiel wird die Blutpumpe ebenfalls in Gegenrichtung betrieben, allerdings nur solange, wie die arterielle Leitung noch Patientenblut enthält.

Vorzugsweise ist die Dialysatpumpe zwangsweise mit der Abfallpumpe zu einer Bilanzierpumpe gekoppelt, beispielsweise mechanisch gekoppelt. Hierdurch wird mechanisch sichergestellt, dass die Dialysatpumpe jeweils exakt dieselbe Menge in Richtung Dialysator pumpt, wie die Abfallpumpe aus Richtung Dialysator abpumpt. Im Falle einer Bilanzierpumpe ist eine separate Ultrafiltrationspumpe vorgesehen, die fluidisch parallel zu der Abfallpumpe angeordnet ist. Die Ultrafiltrationspumpe wird beim Dialysatfüllen der Leitungen im Gegenstrom betrieben, d.h. sie pumpt einen Teil der von der Abfallpumpe stromabwärts gepumpten Flüssigkeit über einen Bypass zurück zur Einlassseite der Abfallpumpe. Hierbei liegt die Pumprate der Ultrafiltrationspumpe stets unter der Pumprate der Abfallpumpe, damit nicht bereits verbrauchtes Dialysat rückwärts in den Dialysator gedrückt werden kann.

Im Folgenden werden zwei Ausführungsbeispiele der Erfindung anhand der Zeichnungen näher erläutert:
Figur 1 zeigt schematisch ein Hämodialysegerät zur Durchführung einer Hämodialyse, wobei beim Dialysatfüllen beide blutseitigen Leitungen mit dem Patienten direkt verbunden bleiben, und
Figur 2 zeigt schematisch das Hämodialysegerät der Figur 1, wobei beim Dialysatfüllen nur die venöse Leitung direkt mit dem Patienten verbunden bleibt, wohingegen die arterielle Leitung über einen Leitungszugang an die venöse Leitung angeschlossen ist.

In den Figuren ist schematisch ein Hämodialysegerät 10 dargestellt, das funktional in eine Dialysatseite 12 und eine Blutseite 14 unterteilt ist. Die Grenze zwischen der Dialysatseite 12 und der Blutseite 14 wird von einer Dialyse-Membran 31 in einem Dialysator 28 gebildet. Die Dialyse-Membran 31 trennt in dem Dialysator 28 eine Dialysatkammer 29 von einer Blutkammer 30.

Die Dialysatseite 12 weist eine Dialysatquelle 16 auf, die das Dialysat für die Dialyse zur Verfügung stellt. Die Dialysatquelle 16 wird von einem Dialysewasser- Tank 17 mit Dialysewasser 18 und einem Additiv-Tank 70 mit einem Dialysat-Additiv 72 gebildet. Dem Additiv-Tank 70 ist eine Additiv-Pumpe 74 nachgeordnet, die das Additiv 72 wohldosiert dem Dialysewasser-Strom zuführt. Es können auch mehrere Additiv-Tanks vorgesehen sein, wobei als Additiv insbesondere Elektrolyte und Puffersubstanzen vorgesehen sind. Das Dialysewasser kann, alternativ zu dem Dialysewasser-Tank 17, auch von einer (nicht dargestellten) Wasseraufbereitungs-Vorrichtung zur Verfügung gestellt werden, in der aus Trinkwasser aus dem öffentlichen Leitungsnetz das Dialysewasser hergestellt wird.

Im Verlauf der Leitung zwischen der Dialysatquelle 16 und der Dialysatpumpe 22 ist eine Dialysat- Durchlaufheizung 47 angeordnet, die das Dialysat derart erwärmt, dass es in dem Dialysator 28 ungefähr Körpertemperatur oder eine geringfügig oberhalb der Körpertemperatur liegende Temperatur hat. Vorliegend ist die Dialysat- Durchlaufheizung 47 in Strömungsrichtung vor dem Additiv-Zulauf angeordnet, so dass die Durchlaufheizung 47 streng genommen nur das Dialysewasser 18 erwärmt, nicht jedoch das fertige Dialysat. Unter einer Dialysat-Durchlaufheizung soll vorliegend jede Durchlaufheizung verstanden werden, die entweder das Dialysewasser oder das fertige Dialysat beim Vorbeifließen erwärmt.

Zwischen der Dialysat-Durchlaufheizung 47 und dem Dialysator 28 ist ein Temperatursensor 50 angeordnet. Ferner ist ein Temperaturregler 96 vorgesehen. Die Dialysat-Durchlaufheizung 47, der Temperatursensor 50 und der Temperaturregler 96 bilden zusammen einen Regelkreis zur Regelung der Dialysat-Temperatur auf eine Solltemperatur. Die Dialysat-Solltemperatur ist so gewählt, dass während des Dialysatfüllens das Dialysat beim Erreichen des Patientenkörpers ungefähr Körpertemperatur hat. Die exakte Regelung der Dialysat-Temperatur ist deshalb sinnvoll und erforderlich, weil das Dialysat beim Dialysatfüllen durch die Dialysator-5 Membran 31 hindurch von der Dialysatseite 12 zu der Blutseite 14 in die extrakorporalen Leitungen 42,44 gepumpt wird, und daher nicht ausgeschlossen werden kann oder sogar erwünscht ist, dass das Dialysat auch schließlich in den Patientenkörper 50 gelangt.

Im weiteren Verlauf der Leitung zwischen der Dialysatpumpe 22 und dem Dialysator 28 ist ein einziger separater Sterilisationsfilter 48 angeordnet, durch den das Dialysat durch Filterung derart "sterilisiert" wird, dass alle Viren, Bakterien, Endotoxine sowie Pilze von ihm zurückgehalten werden. Da die Dialysator-Membran 31 ebenfalls als Sterilisationsfilter wirkt, ist ein zweiter separater Sterilisationsfilter zur Herstellung einer Sterilitäts-Redundanz nicht erforderlich. Um die Sterilisierungseigenschaft des Dialysators 28 sicherzustellen, muss dieser validiert sein und unmittelbar vor Behandlungsbeginn auf Unversehrtheit, das heißt auf Dichtigkeit insbesondere der Membran 31 geprüft sein.

Im Verlauf der Leitung zwischen der Dialysatkammer 29 und der Abfallpumpe 24 ist ein Dialysat- Drucksensor 32 angeordnet, der den Druck der Dialysat-Flüssigkeit in diesem Leitungsabschnitt ermittelt. Parallel zu der Abfallpumpe 24 ist eine Ultrafiltrationspumpe 34 angeordnet, die in beiden Richtungen betrieben werden kann, also im Gegenstrom und im Parallelstrom zu der Abfallpumpe 24 betrieben werden kann.

Von der Dialysatkammer 29 des Dialysators 28 aus verläuft eine Leitung zu einer Abfallpumpe 24, die das Dialysat aus der Dialysatkammer 29 in einen Abfalltank 36 pumpt, in dem das verbrauchte Dialysat 38 gespeichert wird. Alternativ pumpt die Abfallpumpe 24 das Dialysat aus der Dialysatkammer 29 direkt in das öffentliche Abwassernetz, wobei das Dialysat einen Wärmetauscher durchlaufen kann, der das Dialysewasser erwärmt.

Die Dialysatpumpe 22 und die Abfallpumpe 24 sind mechanisch durch eine mechanische Verbindung 26 miteinander verbunden, so dass die Pumpraten der Dialysatpumpe 22 und der Abfallpumpe 24 stets absolut identisch sind. Auf diese Weise bilden die Dialysatpumpe 22 und die Abfallpumpe 24 eine sogenannte Bilanzierpumpe 20. Die mechanische Verbindung 26 kann beispielsweise dadurch realisiert sein, dass die Bilanzierpumpe 20 als Membranpumpe ausgebildet ist, wobei die eine Seite der Pumpenmembran die Membran der Dialysatpumpe 22 und die andere Seite der Pumpenmembran die Membran der Abfallpumpe 24 bildet.

Die Prüfung der Dialysator-Dichtigkeit kann innerhalb des Hämodialysegeräts 10 vor Beginn der eigentlichen Hämodialyse-Behandlung erfolgen. Hierbei wird in der mit Dialysat gefüllten Dialysatkammer 29 mit Hilfe der Pumpen 42,24,34 ein Unterdruck erzeugt, während die Blutkammer 30 ausschließlich Luft enthält. Da die Dialysator-Membran 31 für Luft undurchlässig ist, darf hierbei über einen gewissen Zeitraum kein Druckabfall auf der Dialysatseite 12 durch den Dialysat-Drucksensor 32 festgestellt werden, wenn die Dialysator-Membran 31 unversehrt ist.

Auf der Blutseite der Dialysator-Membran 31 befindet sich die Blutkammer 30 des Dialysators 28, in die eine arterielle Leitung 42 und eine venöse Leitung 44 münden. Im Verlauf der arteriellen Leitung 42 ist eine in beiden Richtungen betreibbare Blutpumpe 40 in Form einer peristaltischen Schlauchpumpe angeordnet, deren Pumprichtung und Pumprate von der Gerätesteuerung 90 gesteuert werden können. Die arterielle Leitung 42 weist an ihrem freien Ende eine arterielle Kanüle 43 und die venöse Leitung 44 an ihrem Ende eine venöse Kanüle 45 auf.

Die Kanülen 43,45 sind mit entsprechenden Kupplungen mit den jeweiligen Leitungsenden verbunden, so dass die Kanülen 43,45 für ihre Entsorgung bzw. zur Vermeidung von Verletzungen unmittelbar nach Abschluss der Behandlung sofort entfernt werden können. Die Kanülen 43,45 dienen der Verbindung der beiden Leitungen 42,44 mit entsprechenden Patienten-Zugängen bzw. Blutgefäßen des Patienten 50. Zwischen der arteriellen Kanüle 43 und der Blutpumpe 40 sind ein arterieller Drucksensor 52 und ein Luftsensor 54 angeordnet, und kann ein Farbsensor und/oder eine automatische Schlauchklemme vorgesehen sein. Über den arteriellen Drucksensor 52 kann der Fluiddruck in der arteriellen Leitung 42 gemessen werden. Der Luftsensor 54 weist zum Einen einen Luftblasendetektor auf, der Luftblasen in der vorbeifließenden Flüssigkeit innerhalb der arteriellen Leitung 42 detektieren kann, und weist zum Anderen eine von der Gerätesteuerung 90 ansteuerbare Leitungsklemme auf, mit der bei Bedarf, und insbesondere bei Detektion von Luft, die arterielle Leitung 42 sofort vollständig geschlossen werden kann.

Im Verlauf der venösen Leitung 44 ist, von dem Dialysator 28 aus betrachtet, zunächst einen Luftfalle 56 vorgesehen, in der Luftblasen der der hindurchfließenden Flüssigkeit zurückgehalten werden. Der Drucksensor kann alternativ auch über eine Luftleitung an die Luftfalle angeschlossen sein. Die Luftfalle 56 ist mit einem separaten Leitungszugang 57 ausgestattet, der in Strömungsrichtung vor der Luftfallenmimik vorgesehen ist, so dass auch die Flüssigkeit, die durch den Leitungszugang 57 eingespeist wird, durch die Luftfalle 57 entlüftet wird.

Zwischen der Luftfalle 56 und der venösen Kanüle 45 sind im Verlauf der venösen Leitung 44 ferner ein venöser Drucksensor 58, ein Luftsensor 60 sowie ein Farbsensor 62 angeordnet. Mit Hilfe des venösen Drucksensors 58 kann der Druck in der venösen Leitung 44 ermittelt werden. Der venöse Luftsensor 60 ist, analog zu dem arteriellen Luftsensor 54, ebenfalls mit einer ansteuerbaren Leitungsklemme ausgestattet. Der Farbsensor 62 ist möglichst nah an der Kanüle 45 angeordnet, und kann die Anwesenheit oder Nichtanwesenheit von Blut in der venösen Leitung 44 optisch detektieren. Der Farbsensor 62 fotometriert hierzu die transparente Leitung 44 bei einer bestimmten charakteristischen Wellenlänge.

Der Dialysator 28 und die blutseitigen Leitungen 42,44 einschließlich der Kanülen 43,45 sind nur für den Einmalgebrauch gedacht, sind also sogenannte Einwegartikel, und als solche Müll bzw. Sondermüll.

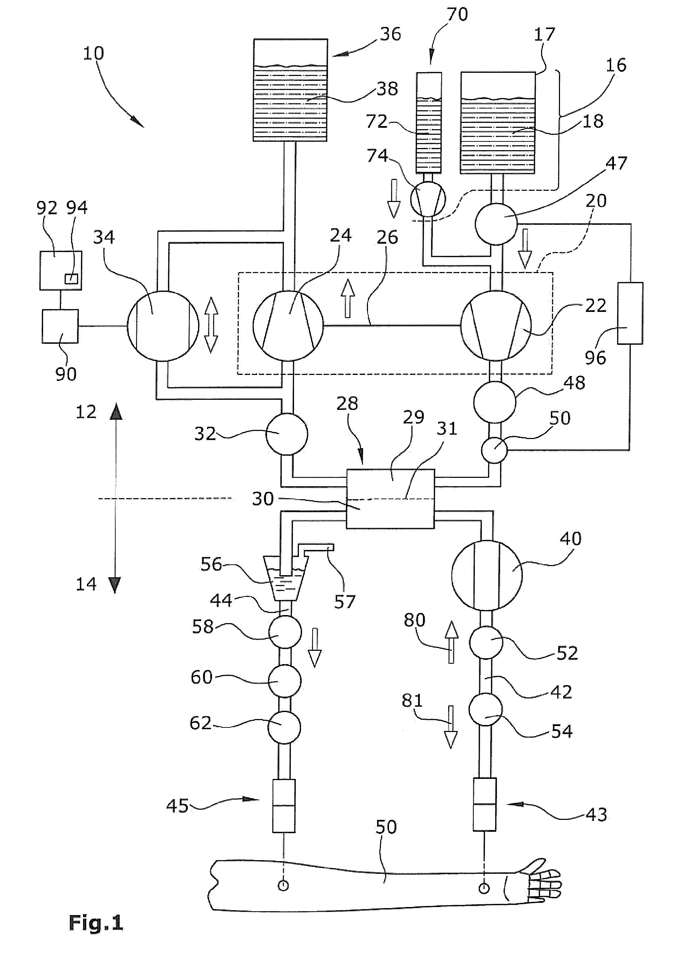

Es ist eine Gerätesteuerung 90 vorgesehen, die u.a. den Betrieb der Ultrafiltrationspumpe 34 steuert. Die Gerätesteuerung 90 ist ferner mit einem Touchscreen 92 verbunden, auf dem während des Dialysatfüllens ein Anwesenheitstaster 94 dargestellt und funktional eingerichtet ist. Der Anwesenheitstaster 94 ist nur bei dem in der Figur 1 dargestellten ersten Ausführungsbeispiel erforderlich, falls in der arteriellen Leitung kein Luftsensor vorhanden ist, kann jedoch grundsätzlich bei beiden Ausführungsbeispielen vorgesehen sein.

Während der Hämodialyse pumpt die Blutpumpe 40 in Betriebsrichtung 80 Patientenblut aus dem Patientenkörper 50 durch die arterielle Leitung 42 in Richtung Dialysator 28, in dem das Patientenblut an der Dialysator-Membran 31 vorbei in die venöse Leitung 44 und zurück in den Patientenkörper 50 fließt. Gleichzeitig pumpt die Bilanzierpumpe 20 Dialysat durch die Dialysatkammer 29 des Dialysators 28. Wenn die eigentliche Hämodialyse, also der Vorgang der Blutwäsche, beendet ist, beginnt das sogenannte Ablegen, also die Trennungsvorbereitung und die Trennung des Hämodialysegeräts von dem Patienten. Danach können die desinfizierenden Maßnahmen und gegebenenfalls die Vorbereitungen für die folgende Hämodialyse-Behandlung des folgenden Patienten vorgenommen werden. Die Trennungsvorbereitung besteht im Wesentlichen aus dem Füllen der extrakorporalen Leitungen 42,44 mit Dialysat, um das Patientenblut möglichst vollständig aus den beiden Leitungen 42,44 zurück in den Patienten 50 zu pumpen.

In der Figur 1 ist ein erstes Ausführungsbeispiel für die Trennungsvorbereitung bzw. das Dialysatfüllen dargestellt. Hierbei bleiben die beiden Kanülen 43,45 der beiden extrakorporalen Leitungen 42,44 direkt und unmittelbar an den Patienten 50 angeschlossen. Ein Umstecken der extrakorporalen Leitungen 42,44 ist also nicht erforderlich.

Sobald das Dialysatfüllen von der Gerätesteuerung 90 ausgelöst ist, werden zunächst die Blutkammer 30 des Dialysators 28, die arterielle Leitung 42 und die venöse Leitung 44 mit Dialysat gefüllt, wodurch das Patientenblut aus beiden Leitungen 42,44 zurück in den Patienten 50 geschoben wird. Hierbei ist die Bilanzierpumpe 20 in Betrieb, so dass die Dialysatpumpe 22 Dialysat in Richtung Dialysator 28 pumpt, während gleichzeitig die Ultrafiltrationspumpe 34 im Gegenstrom arbeitet. Hierdurch wird ein Teil oder das gesamte von der Dialysatquelle 16 kommende und durch die Dialysatpumpe 22 gepumpte Dialysat durch die Dialysator-Membran 31 hindurchgepresst, und zwar mit einer Durchtrittsrate, die genau der im Gegenstrom betriebenen Pumprate der Ultrafiltrationspumpe 34 entspricht. Hierbei wird die Blutpumpe 40 in Gegenrichtung 81 und mit ungefähr der halben Pumprate der Ultrafiltrationspumpe 34 betrieben. Die Pumprate der Ultrafiltrationspumpe 34 ist so gewählt, dass das Dialysat an den Enden der beiden Leitungen 42,44 etwa gleichzeitig ankommt. Die Pumprate der Ultrafiltrationspumpe 34 ergibt sich also aus dem Verhältnis der Innenvolumina der arteriellen Leitung 42 und der venösen Leitung 44.

Das von der Dialysatquelle 16 aus kommende Dialysat wird in der Dialysat- Durchlaufheizung 47 auf eine Solltemperatur aufgeheizt und auf dem Weg zum Dialysator 28 in dem separaten Sterilisationsfilter 48 gefiltert. Die Dialysat-Temperatur wird durch einen Temperaturregler 96 und mit Hilfe des Temperatursensors 50 und der Durchlaufheizung 47 auf eine konstante Solltemperatur geregelt, die in der Nähe von 37°C bzw. in der Regel wenige Grad über der Körpertemperatur liegt.

Die beiden Leitungen 42,44 werden so weit mit Dialysat gefüllt, bis annähernd kein Patientenblut mehr in den Leitungen 42,44 vorhanden ist. Das Füllen der Leitungen 42,44 mit Dialysat erfolgt volumengesteuert. Das Dialysat-Gesamtfüllvolumen, mit dem die Blutseite 14 aufgefüllt wird, kann größer sein als das Hohlraumvolumen der Dialysator-Blutkammer 30 und der beiden daran angeschlossenen Leitungen 42,44 zusammen. Auf diese Weise wird sichergestellt, dass das gesamte Patientenblut der Blutseite zurück in den Patienten 50 geschoben wird, wobei auch eine kleine Menge Dialysat auf diese Weise in den Patienten 50 gepumpt wird.

Neben der Volumensteuerung mit einem vorgegebenen Gesamtfüllvolumen wird das Füllen der Blutseite 14 mit Dialysat durch den arteriellen Drucksensor 52 und den venösen Drucksensor 58 überwacht.

Sobald der von den beiden vorgenannten Drucksensoren 52,58 gemessene Druck eine festgelegte Dialysatdurchtritts- Druckschwelle unterschreitet, wird das Dialysatfüllen volumengesteuert beendet, das heißt, es wird noch ein geringes Restvolumen Dialysat zur Blutseite 14 gepumpt, und danach das Dialysatfüllen beendet. Alternativ oder ergänzend hierzu kann auch der von dem Dialysat-Drucksensor 32 gemessene Druck hierfür überwacht werden.

Die Viskosität von Patientenblut ist höher als die Viskosität von Dialysat, so dass der effektive Strömungswiderstand des Patientenblutes beim Durchtritt durch die Kanülen 43,45 in den Patienten 50 höher ist als der effektive Strömungswiderstand des Dialysats. Sobald also nicht mehr Patientenblut, sondern Dialysat durch die Kanülen 43,45 in den Patienten 50 strömt, sinkt der Strömungswiderstand an den Kanülen 43,45, so dass der von den Drucksensoren 32, 52,58 gemessene Druck unter die entsprechend festgelegte Dialysatdurchtritts-Druckschwelle fällt. Hierdurch wird eine Beendigung des Dialysatfüllens ausgelöst, indem noch ein festgelegtes Sicherheits-Restvolumen Dialysat zur Blutseite 14 gepumpt wird, und danach das Dialysatfüllen zwangsweise beendet wird. Das Dialysatfüllen kann danach noch manuell fortgesetzt werden, um Blutreste aus den extrakorporalen Leitungen 42,44 bzw. den Kanülen 43,45 noch in den Patienten 50 pumpen zu können.

Bei der Volumensteuerung wird grundsätzlich aus den aufaddierten Pumpzyklen der beteiligten Pumpe(n), vorliegend also aus den Pumpzyklen der Ultrafiltrationspumpe 34, auf das zur Blutseite gepumpte Dialysatvolumen geschlossen.

Während des gesamten Dialysatfüllens muss der Anwesenheitstaster 94 von einer Bedienperson gedrückt werden. Sobald und wenn der Anwesenheitstaster 94 nicht gedrückt ist, stoppt die Gerätesteuerung das Dialysatfüllen. Hintergrund hierfür ist der Umstand, dass bei dem ersten Ausführungsbeispiel die arterielle Leitung 42 in zwei Flussrichtungen betrieben wird, nämlich während der eigentlichen Dialyse in Betriebsrichtung 80 und während des Dialysatfüllens in der Gegenrichtung 81. Aus diesem Grund kann in der arteriellen Leitung 42 keine Luftfalle vorgesehen werden, die prinzibedingt nur unidirektional betrieben werden kann. Da in der arteriellen Leitung 42 keine Luftfalle eingebaut werden kann, kann es notwendig sein, die Luftfreiheit der Flüssigkeit in der arteriellen Leitung 42 während des Dialysatfüllens durch eine Bedienperson überwachen zu lassen, insbesondere dann, wenn in der arteriellen Leitung kein Luftsensor vorgesehen sein sollte. Die Anwesenheit der Bedienperson wird durch den Anwesenheitstaster 94 sichergestellt.

Sobald das Dialysatfüllen abgeschlossen ist, werden die arterielle und die venöse Leitung 42,44 von dem Patienten 50 abgenommen, werden also die beiden Kanülen 43,45 von den jeweiligen Zugängen bzw. den betreffenden Blutgefäßen des Patienten 50 getrennt.

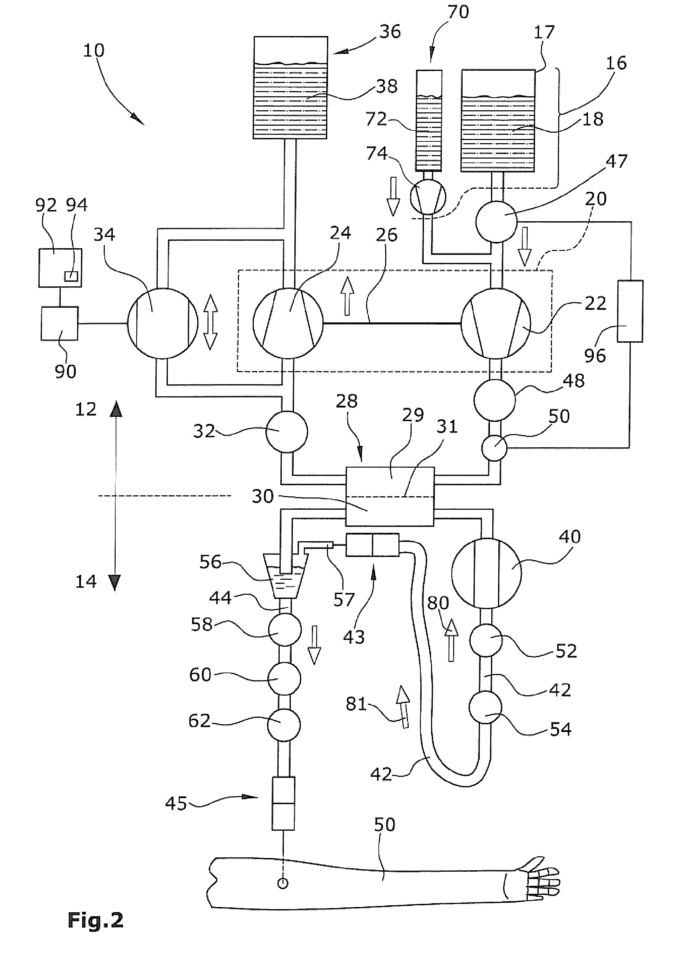

Bei dem in der Figur 2 dargestellten zweiten Ausführungsbeispiel wird vor dem Starten des Dialysatfüllens die arterielle Kanüle 43 bzw. das betreffende Leitungsende an den separaten Leitungszugang 57 der Luftfalle 56 angeschlossen, so dass anschließend die Flüssigkeit aus der arteriellen Leitung 42 in die venöse Leitung 44 eingeleitet werden kann. Auf diese Weise wird sichergestellt, dass auch die Flüssigkeit aus der arteriellen Leitung eine Luftfalle durchlaufen muss, bevor sie zu dem Patientenkörper 50 geleitet wird. Die Blutpumpe 40 arbeitet auch im zweiten Ausführungsbeispiel in Gegenrichtung 81, wobei sie zunächst den gesamten Dialysatstrom durch die Dialysator- Membran 31 aus der Blutkammer 30 abpumpt, so dass zunächst das gesamte Patientenblut aus der arteriellen Leitung 42 durch den Leitungszugang 57 der Luftfalle 56 in die venöse Leitung 44 gepumpt wird. Sobald die arterielle Leitung 42 nur noch Dialysat enthält, wird die Blutpumpe 40 angehalten und wird anschließend das gesamte durch die Dialysator-Membran 31 hindurchtretende Dialysat in die venöse Leitung 44 gepumpt. Neben einer Volumensteuerung der gesamten Dialysatfüllungs- Prozedur wird auch hierbei die Dialysatankunft an der venösen Kanüle 45 durch die Überwachung des Fluiddrucks durch den venösen Drucksensor 58 und die Überwachung der Flüssigkeitsfarbe bzw. -transmissivität durch den Farbsensor 62 detektiert.

Sobald das Dialysatfüllen abgeschlossen ist, wird die venöse Leitung 44 von dem Patientenkörper 50 und die arterielle Leitung 42 von dem Leitungszugang 57 abgenommen. Die beiden Kanülen 43,45, die mit Kupplungen an den Leitungen 42,44 befestigt sind bzw. waren, werden ggf. von den Leitungen abgekoppelt und entsorgt, so dass von den Kanülen 43,45 keine Verletzungsgefahr mehr ausgeht.

## Patentansprüche

1. Hämodialysegerät (10) umfassend
eine Dialysatseite (12) mit einer Dialysatkammer (29) eines eine Membran (31) aufweisenden Dialysators (28), einer Dialysatpumpe (22), die Dialysat von einer Dialysatquelle (16) zu der Dialysatkammer (29) pumpt, einer Dialysat- Durchlaufheizung (47) vor der Dialysatkammer (29), und einer Abfallpumpe (24), die das Dialysat von der Dialysatkammer (29) weg pumpt,
eine Blutseite (14) mit einer arteriellen Leitung (42), einer Blutpumpe (40), einer Blutkammer (30) des Dialysators (28) und einer venösen Leitung (44), und
eine Gerätesteuerung (90), die dazu eingerichtet ist, ein Verfahren zum Beenden einer Hämodialyse- Behandlung eines Patienten (50) durchzuführen,
mit dem Verfahrensschritt:
Füllen der an den Patienten (50) angeschlossenen arteriellen und venösen Leitung (42,44) mit Dialysat, das von der Dialysatpumpe (22) durch die Dialysator- Membran (31) in die arterielle und die venöse Leitung (42,44) gepumpt wird, bis kein Patientenblut mehr in den Leitungen (42, 44) vorhanden ist,
wobei während des Dialysatfüllens das von der Dialysatpumpe (22) zu der Dialysatkammer (29) gepumpte Dialysat durch die Dialysat-Durchlaufheizung (47) auf eine Solltemperatur aufgeheizt wird,**dadurch gekennzeichnet**, dasswährend des Dialysatfüllens eine ständige Bestimmung des Fluiddrucks durch einen Dialysat-Drucksensor (32) zwischen der Dialysatkammer (29) und der Abfallpumpe (24), einen arteriellen Drucksensor (52) oder einen venösen Drucksensor (58) vorgenommen wird, wobei das Dialysatfüllen volumengesteuert beendet wird, sobald der gemessene Fluiddruck eine festgelegte Dialysatdurchtritts-Druckschwelle durchschreitet.

2. Hämodialysegerät (10) nach Anspruch 1, wobei in einer Dialysatleitung zwischen der Dialysat- Durchlaufheizung (47) und dem Dialysator (28) ein Temperatursensor (50) angeordnet ist, das Hämodialysegerät (10) einen Temperaturregler (96) aufweist, und der Temperaturregler (96) während des Dialysatfüllens die Temperatur des Dialysats mit Hilfe des Temperatursensors (50) und der Dialysat-Durchlaufheizung (47) regelt.

3. Hämodialysegerät (10) nach einem der vorangegangenen Ansprüche, wobei im Verlauf der venösen Leitung (44) eine Luftfalle (56) und ein separater Leitungszugang (57) vor oder an der Luftfalle (56) vorgesehen ist, wobei vor dem Starten des Dialysatfüllens das patientenseitige Ende der arteriellen Leitung (42) an den Leitungszugang (57) angeschlossen wird.

4. Hämodialysegerät (10) nach Anspruch 1 oder 2, wobei im Verlauf der arteriellen Leitung (44) und im Verlauf der venösen Leitung (42) jeweils ein Luftsensor (60,54) angeordnet ist, wobei während des Dialysatfüllens beide Leitungen (44,42) jeweils mit einer Kanüle (45,43) unmittelbar mit dem Patienten (50) verbunden sind.

5. Hämodialysegerät (10) nach Anspruch 1 oder 2, wobei ein mit der Gerätesteuerung (90) verbundener Anwesenheitsdetektor (94) vorgesehen ist, wobei die Gerätesteuerung (90) das Dialysatfüllen sofort stoppt, wenn der Anwesenheitsdetektor (94) nicht gedrückt ist.

6. Hämodialysegerät (10) nach einem der vorangegangenen Ansprüche, wobei das Dialysatfüllen volumengesteuert erfolgt, wobei das Dialysat-Füllvolumen größer ist als das Volumen der Dialysator- Blutkammer (30) zuzüglich der Volumina der beiden Leitungen (42,44).

7. Hämodialysegerät (10) nach einem der vorangegangenen Ansprüche, wobei zwischen der Dialysatquelle (16) und der Dialysator-Dialysatkammer (29) ein einziger Sterilisationsfilter (48) vorgesehen ist.

8. Hämodialysegerät (10) nach einem der vorangegangenen Ansprüche, wobei im Verlauf der venösen Leitung (44) ein Farbsensor (62) angeordnet ist, wobei das Dialysatfüllen volumengesteuert beendet wird, sobald der Farbsensor (62) in der venösen Leitung (44) Dialysat detektiert.

9. Hämodialysegerät (10) nach einem der vorangegangenen Ansprüche, wobei während des Dialysatfüllens die Blutpumpe (40) ungefähr mit der Pumprate betrieben wird, die eine ungefähr gleichzeitige Ankunft des Dialysats an beiden Enden der Leitungen (42,44) gewährleistet.

10. Hämodialysegerät (10) nach einem der vorangegangenen Ansprüche, wobei die Dialysatpumpe (22) zwangsweise mit der Abfallpumpe (24) zu einer Bilanzierpumpe (20) gekoppelt ist, und eine separate Ultrafiltrationspumpe (34) fluidisch parallel zu der Abfallpumpe (24) vorgesehen ist, wobei die Ultrafiltrationspumpe (34) beim Dialysatfüllen im Gegenstrom arbeitet.

## Claims

1. A haemodialysis device (10) comprising
a dialysate side (12) comprising a dialysate chamber (29) of a dialyzer (28) having a membrane (31), a dialysate pump (22) pumping dialysate from a dialysate source (16) to the dialysate chamber (29), a dialysate continuous-flow heater (47) upstream of the dialysate chamber (29), and a waste pump (24) pumping the dialysate away from the dialysate chamber (29),
a blood side (14) comprising an arterial line (42), a blood pump (40), a blood chamber (30) of the dialyzer (28) and a venous line (44), and
a device control (90) configured to perform a method for ending a haemodialysis treatment of a patient (50),
comprising the method step:
filling the arterial and venous lines (42, 44), which are connected with the patient (50), with dialysate which is pumped by the dialysate pump (22) through the dialyzer membrane (31) into the arterial and venous lines (42, 44) until no more patient blood is present in the lines (42, 44),
wherein, while dialysate is filled in, the dialysate pumped to the dialysate chamber (29) by the dialysate pump (22) is heated to a set temperature by the dialysate continuous-flow heater (47), **characterized in that**, while dialysate is filled in, a continuous determination of the fluid pressure is performed by a dialysate pressure sensor (32) between the dialysate chamber (29) and the waste pump (24), an arterial pressure sensor (52) or a venous pressure sensor (58), wherein filling the dialysate is ended in a volume-controlled manner, as soon as the fluid pressure measured passes a defined dialysate passage pressure threshold.

2. The haemodialysis device (10) of claim 1, wherein a temperature sensor (50) is arranged in a dialysate line between the dialysate continuous-flow heater (47) and the dialyzer (28), the haemodialysis device (10) comprises a temperature control (96), and the temperature control (96) controls the temperature of the dialysate by means of the temperature sensor (50) and the dialysate continuous-flow heater (47) while the dialysate is filled in.

3. The haemodialysis device (10) of one of the preceding claims, wherein an air trap (56) is provided along the venous line (44) and a separate line port (57) is provided upstream of or at the air trap (56), wherein prior to beginning the dialysate filling the patient-side end of the arterial line (42) is connected to the line port (57).

4. The haemodialysis device (10) of claim 1 or 2, wherein an air sensor (60, 54) is arranged along the arterial line (44) and along the venous line (42), respectively, wherein, while the dialysate is filled in, both lines (44, 42) are respectively connected directly with the patient (50) via a cannula (45, 43).

5. The haemodialysis device (10) of claim 1 or 2, wherein a presence detector (94) is provided that is connected with the device control (90), the device control (90) stopping the dialysate filling immediately if the presence detector (94) is not pushed.

6. The haemodialysis device (10) of one of the preceding claims, wherein the dialysate filing is performed in a volume-controlled manner, the dialysate filling volume being greater than the volume of the dialyzer blood chamber (30) plus the volumes of the two lines (42, 44).

7. The haemodialysis device (10) of one of the preceding claims, wherein a single sterilization filter (48) is provided between the dialysate source (16) and the dialyzer chamber (29).

8. The haemodialysis device (10) of one of the preceding claims, wherein a color sensor (62) is arranged along the venous line (44), the dialysate filling being ended in a volume-controlled manner as soon as the color sensor (62) detects dialysate in the venous line (44).

9. The haemodialysis device (10) of one of the preceding claims, wherein, while the dialysate is filled in, the blood pump (40) is operated at approximately the pumping rate that ensures an approximately simultaneous arrival of the dialysate at both ends of the lines (42, 44).

10. The haemodialysis device (10) of one of the preceding claims, wherein the dialysate pump (22) is forcibly coupled with the waste pump (24) to form a balancing pump (20), and wherein a separate ultrafiltration pump (34) is provided fluidically in parallel with the waste pump (24), the ultrafiltration pump (34) operating in a counter current mode while the dialysate is filled in.

## Revendications

1. Appareil d'hémodialyse (10) comprenant
un côté dialysat (12) avec une chambre à dialysat (29) d'un dialyseur (28) comprenant une membrane (31), une pompe à dialysat (22) pompant de dialysat d'une source de dialysat (16) vers la chambre à dialysat (29), un chauffage de dialysat instantané (47) en amont de la chambre à dialysat (29), et une pompe à déchets (24) pompant le dialysat loin de la chambre à dialysat (29),
un côté sang (14) avec un conduit artériel (42), une pompe à sang (40), une chambre à sang (30) du dialyseur (28) et un conduit veineux (44), et
une commande d'appareil (90) configurée pour exécuter un procédé pour terminer un traitement de dialyse d'un patient (50),
avec l'étape de procédé suivante:
remplir les conduits artériels et veineux (42, 44), connectés au patient (50), avec dialysat pompé dans les conduits artériels et veineux (42, 44) par la pompe à dialysat (22) à travers la membrane (31) du dialyseur jusqu'à ce qu'aucun sang du patient ne soit présent dans les conduits (42, 44),
pendant le remplissage de dialysat, le dialysat pompé par la pompe à dialysat (22) vers la chambre à dialysat (29) étant chauffé à une température de consigne par le chauffage de dialysat instantané (47), **caractérisé en ce que**, pendant le remplissage de dialysat, la pression de fluide est continûment déterminée par un capteur de pression de dialysat (32) entre la chambre à dialysat (29) et la pompe à déchets (24), un capteur de pression artérielle (52) ou un capteur de pression veineuse (58), le remplissage de dialysat étant terminé en fonction du volume au moment que la pression de fluide mesurée passe un seuil de pression défini pour la passage du dialysat.

2. Appareil d'hémodialyse (10) selon la revendication 1, dans lequel un capteur de température (50) est disposé dans un conduit à dialysat entre le chauffage de dialysat instantané (47) et le dialyseur (28), l'appareil d'hémodialyse (10) comprend un régulateur de température (96), et le régulateur de température (96) règle la température du dialysat par moyen du capteur de température (50) et le chauffage de dialysat instantané (47) pendant le remplissage de dialysat.

3. Appareil d'hémodialyse (10) selon l'une quelconque des revendications précédentes, dans lequel, dans le parcours du conduit veineux (44), une piège à air (56) est prévu et un port d'accès au conduit (57) séparé est prévu en amont de ou à ladite piège à air (56), l'extrémité du conduit artériel (42), côté patient, étant raccordée avec le port d'accès du conduit (57) avant le commencement du remplissage de dialysat.

4. Appareil d'hémodialyse (10) selon la revendication 1 ou 2, dans lequel un capteur d'air (fiv, 54) est disposé respectivement dans le parcours du conduit artériel (44) et dans le parcours du conduit veineux (42), et dans lequel, pendant le remplissage de dialysat, chaque conduit (44, 42) est connecté directement avec le patient (50) respectivement par une canule (45, 43).

5. Appareil d'hémodialyse (10) selon la revendication 1 ou 2, dans lequel un détecteur de présence (94) est prévu, qui est relié à la commande d'appareil (90), la commande d'appareil (90) terminant le remplissage de dialysat immédiatement si le détecteur de présence (94) n'a pas été poussé.

6. Appareil d'hémodialyse (10) selon l'une quelconque des revendications précédentes, dans lequel le remplissage de dialysat est effectué en fonction du volume, le volume de remplissage de dialysat étant plus grand que le volume de la chambre à sang (30) du dialyseur plus les volumes des deux conduits (42, 44).

7. Appareil d'hémodialyse (10) selon l'une quelconque des revendications précédentes, dans lequel un seul filtre de stérilisation (48) est prévu entre la source de dialysat (16) et la chambre à dialysat (29) du dialyseur.

8. Appareil d'hémodialyse (10) selon l'une quelconque des revendications précédentes, dans lequel un capteur de couleurs (62) est disposé dans le parcours du conduit veineux (44), le remplissage de dialysat étant terminé en fonction du volume au moment que le capteur de couleurs (62) détecte de dialysat dans le conduit veineux (44).

9. Appareil d'hémodialyse (10) selon l'une quelconque des revendications précédentes, dans lequel, pendant le remplissage de dialysat, la pompe à sang (40) est opérée avec environ le débit de pompage qui garantit une arrivée environ simultanée du dialysat à les deux extrémités des conduits (42, 44).

10. Appareil d'hémodialyse (10) selon l'une quelconque des revendications précédentes, dans lequel, par couplage force, la pompe à dialysat (22) et la pompe à déchets forment une pompe d'équilibrage (20), et une pompe d'ultrafiltration (34) séparée est prévue de manière fluidiquement en parallèle à la pompe à déchets (24) la pompe d'ultrafiltration (34) opérant en contrecourant lors du remplissage de dialysat.
